# FASCICULE DE BREVET EUROPEEN

(11) **EP 3 420 892 B1**
(45) Date de publication et mention de la délivrance du brevet: **08.05.2024**
(21) Numéro de dépôt: 18178241.8
(22) Date de dépôt: 11.09.2007
(51) Int. Cl.: A61B 5/00

(54) **CABINE DE SANTÉ**
TELEMEDIZIN-KABINE
HEALTH CABIN

(43) Date de publication de la demande: 02.01.2019
(62) Demande divisionnaire de: 07823802.9
(73) Titulaire: H4D, 13100 Aix-en-Provence (FR)
(72) Inventeur: BAUDINO, Franck, 75016 Paris (FR); BAUDINO, Laurent, 75016 Paris (FR)
(74) Mandataire: Cabinet Beau de Loménie

(56) Documents cités:
- US-A- 5 680 871
- US-A1- 2004 125 996
- US-A1- 2005 101 884
- US-A1- 2005 113 650

## Description

### Arrière-plan de l'invention

L'invention concerne une cabine de santé destinée à obtenir des mesures permettant d'établir un bilan de santé d'un utilisateur ou d'une population.

De façon connue, l'établissement d'un bilan de santé nécessite la prise préalable d'une pluralité de mesures (poids, pouls, température, etc.) sous la supervision d'un personnel médical.

Dans certains cas, ces opérations doivent être répétées à intervalles réguliers pour suivre l'évolution des mesures dans le temps.

La présence obligatoire d'un personnel de santé pose un certain nombre de problèmes.

Tout d'abord, dans les pays émergeants, où la densité des personnels de santé est faible, on comprend aisément qu'il est difficile de mener une campagne de santé, notamment en urgence, lorsqu'un risque épidémiologique se présente.

Dans les autres régions, le principal frein à l'établissement de bilans de santé individuels ou collectifs à grande échelle est le coût relativement élevé induit par la présence du personnel de santé précité.

Afin de limiter les problèmes précités, on connaît, notamment par le document US 5 544 649, des méthodes dites "de télémédecine" dans lesquelles les patients communiquent avec un médecin ou un personnel de santé à distance, via un réseau de télécommunications, les patients effectuant eux-mêmes un certain nombre de mesures transmises au médecin via le réseau.

On notera en tout premier lieu que ces solutions ne sont pas vraiment satisfaisantes puisqu'elles nécessitent la présence, certes à distance, d'un personnel de santé au moment des mesures.

Une solution qui vient naturellement à l'esprit pourrait consister à faire effectuer les mesures par le patient lui-même, par exemple à son domicile, hors de tout contact avec un personnel médical, puis à transmettre les résultats de ces mesures à un centre distant pour un traitement ultérieur.

Mais une telle pratique est difficilement envisageable, car il est connu que les résultats de mesures médicales sont fortement liés aux conditions dans lesquelles sont effectuées les prises de mesures, et notamment à l'état de stress ou de fatigue du patient et aux conditions environnementale météorologiques, sonores et lumineuses au moment de la prise de mesure.

Autrement dit, sans la connaissance de ces informations, les résultats des mesures ne seraient pas exploitables par le médecin avec une fiabilité suffisante.

US 2005/113650 révèle un kiosque avec de différents capteurs de paramètres physiologiques et environnementaux. Ces paramètres peuvent être manipulés pour déterminer une condition physiologique de l'utilisateur.

### Objet et résumé de l'invention

La présente invention vise principalement à résoudre les inconvénients précités.

A cet effet, l'invention concerne une cabine de santé comportant une coque, au moins un fauteuil et au moins un moyen de mesure d'une donnée relative à la santé d'un utilisateur. Cette cabine comporte :
- des moyens pour déterminer, au moment d'une prise de mesure, au moins une condition de la prise de mesure ; et
- des moyens pour enregistrer, dans une structure de données, le résultat de cette prise de mesure en association avec la condition précitée.

Au sens de l'invention, l'expression "cabine de santé" doit être interprétée de façon large, et désigne tout local délimité par une coque, dans lequel un utilisateur peut pratiquer par lui-même un certain nombre de mesures relatives à sa santé sans que la présence d'un personnel médical soit nécessaire, même à distance.

Une cabine de santé au sens de l'invention peut par exemple être transportable pour pouvoir être installée temporairement à un endroit donné. Des cabines de santé au sens de l'invention peuvent notamment être matérialisées par des "mobiles homes" ou par des camions de santé du type de ceux que l'on connaît pour la collecte du sang.

Une cabine de santé au sens de l'invention peut aussi être constituée par une structure fixe destinée à être installée dans des lieux de passage, par exemple dans des aéroports ou dans des hôtels.

Ce qui différencie les cabines de santé selon l'invention des installations connues à ce jour réside dans le fait que les conditions environnementales au moment d'une prise de mesure sont enregistrées et associées dans une structure de données avec le résultat de cette mesure.

Ainsi, lorsque le personnel médical prend connaissance des résultats de mesure, il connaît parfaitement les conditions dans lesquelles ces mesures ont été prises, ce qui rend ces données parfaitement exploitables.

Les moyens de détermination précités sont aptes à mesurer au moins une donnée parmi :
- une position de l'utilisateur ;
- la température à l'intérieur et/ou à l'extérieur de la coque ;
- un niveau sonore à l'intérieur et/ou à l'extérieur de la coque ;
- un taux d'humidité à l'intérieur et/ou à l'extérieur de la coque ; et
- un niveau de luminosité à l'intérieur et/ou à l'extérieur de la coque.

Il est en effet connu que les données suivantes sont nécessaires à une bonne interprétation d'au moins une mesure médicale.

En particulier, la position de l'utilisateur influe fortement sur la mesure de sa tension artérielle. Il est recommandé d'effectuer ces mesures en position assise, le bras reposant sur une table et le brassard gonflable positionné sur ce bras au niveau du coeur.

Il est connu également que les mesures de tension artérielle sont fortement influées par les événements favorisant une augmentation des chiffres tensionnels, à savoir notamment l'exposition au froid, au bruit et des efforts physiques.

Il est également connu que le stress environnemental et l'hyperthermie influent fortement sur le nombre de pulsations cardiaques par minute.

Il est également connu que la mesure de saturométrie par oxygénation sanguine est fortement perturbée par la luminosité ambiante et par le taux d'humidité au niveau de la peau.

D'une façon générale, les moyens de détermination aptes à mesurer les données précitées peuvent être constitués par des capteurs appropriés associés à des moyens de calcul.

Par exemple, pour s'assurer qu'un utilisateur est assis confortablement au moment d'une prise de tension artérielle, il est envisageable d'utiliser des capteurs de pression placés sur le siège et sur le dossier du fauteuil de la cabine.

Pour s'assurer que son bras repose convenablement, des capteurs peuvent être positionnés sur l'accoudoir du fauteuil de la cabine.

Pour mesurer la température, le niveau sonore, le taux d'humidité et le niveau de luminosité à l'intérieur ou à l'extérieur de la coque, on pourra utiliser un thermomètre, un microphone, un hygromètre et une cellule photoélectrique.

La cabine de santé comporte des moyens pour contrôler que la mesure de santé est effectuée lorsqu'une condition répond au moins à un critère prédéterminé.

Cette caractéristique permet d'effectuer les mesures de santé dans des conditions optimales.

Par exemple, on pourra décider de ne mesurer la tension artérielle que lorsque le patient est en position assise depuis au moins cinq minutes.

Dans un mode de réalisation qui n'est pas couvert par les revendications, ces moyens de contrôle sont optionnels, puisqu'il peut être important, notamment suite à une catastrophe naturelle, d'effectuer des mesures de santé en grand nombre, pour prendre des mesures d'urgence, même lorsque les conditions de mesure ne sont pas optimales.

Dans d'autres modes de réalisation, les moyens de contrôle pourront être systématiquement mis en oeuvre.

Dans un mode préféré de réalisation, la coque de la cabine de santé selon l'invention comporte au moins une ouverture permettant de relier cette cabine de santé à au moins une autre cabine de santé.

On forme ainsi un "hôpital" constitué par une pluralité de cabines de santé qui communiquent entre elles.

Cette caractéristique s'avère particulièrement avantageuse lorsqu'il s'agit de garantir certaines conditions prédéterminées au sein de plusieurs cabines de santé installées dans une même zone.

Par exemple, dans une région particulièrement chaude, il est plus facile d'abaisser la température dans plusieurs cabines de santé qui communiquent entre elles que dans le même nombre de cabines de santé prises individuellement.

La cabine de santé selon l'invention comporte des moyens de transmission de la structure de données à un centre distant.

Cette caractéristique permet avantageusement de faciliter la collecte des structures de données relevées dans une pluralité de cabines de santé ou dans un hôpital au sens de l'invention.

Dans un mode de réalisation particulier, la cabine de santé selon l'invention comporte des moyens d'alimentation en énergie.

Ces moyens d'alimentation en énergie peuvent par exemple fonctionner à base d'énergie solaire, ou de cycles thermodynamiques ouverts/et/ou fermés. Ils permettent d'alimenter la cabine de santé en électricité et de réguler la température à l'intérieur de la cabine.

Pour plus de détails sur de tels moyens d'alimentation en énergie, l'homme du métier pourra se reporter aux documents de brevet FR 2 462 584 et FR 2 588 645.

### Brève description des dessins

D'autres caractéristiques et avantages de la présente invention ressortiront de la description faite ci-dessous, en référence aux dessins et à l'annexe 1 annexés qui en illustrent un exemple de réalisation dépourvu de tout caractère limitatif. Sur les figures :
- la figure 1 représente une cabine de santé conforme à l'invention dans un mode particulier de réalisation ;
- la figure 2 représente les connexions d'un ordinateur utilisé dans la cabine de santé de la figure 1 ;
- la figure 3 représente plusieurs cabines de santé conformes à l'invention reliées entre elles pour former un hôpital ; et
- l'annexe 1 représente un exemple de structure de données générée par la cabine de santé de la figure 1.

### Description détaillée d'un mode de réalisation

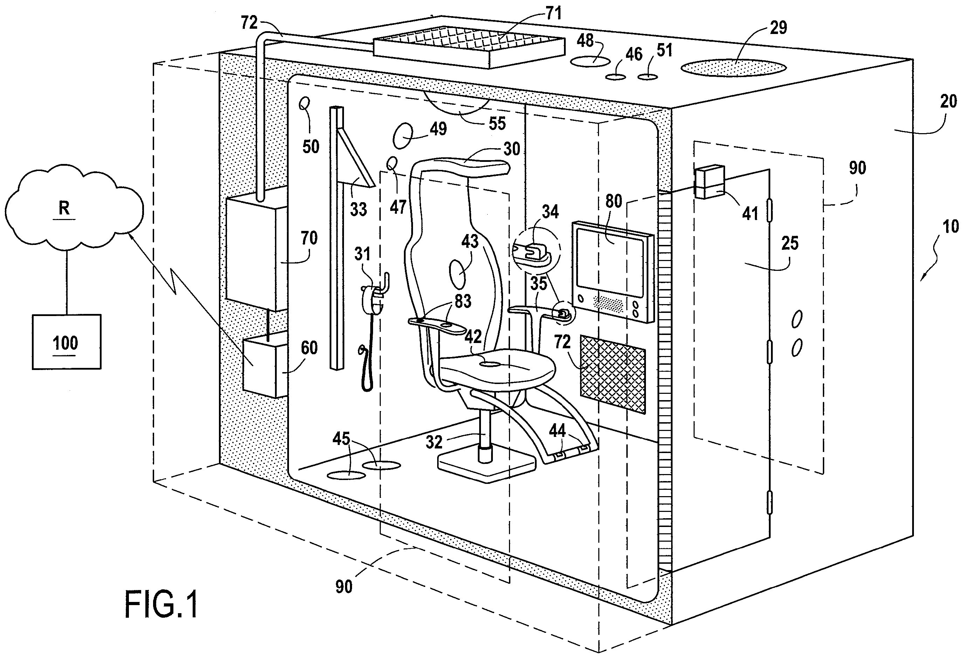

La **figure 1** représente une cabine de santé 10 conforme à l'invention dans un mode particulier de réalisation.

La cabine de santé 10 comporte principalement une coque 20 et un fauteuil 30.

Dans un autre mode de réalisation, la cabine de santé 10 conforme à l'invention peut comporter plusieurs fauteuils 30.

La cabine de santé 10 comporte plusieurs moyens de mesure de données relatives à la santé d'un utilisateur.

Dans l'exemple de réalisation décrit ici, ces moyens de mesure sont constitués par :
- un brassard gonflable 31 pour mesurer la tension artérielle au niveau du bras d'un utilisateur, ce brassard comportant en outre des capteurs de pulsations cardiaques non représentés ;
- une balance 32 pour mesurer le poids de l'utilisateur lorsque celui-ci est assis sur le fauteuil 30 ;
- une toise 33 pour mesurer l'utilisateur en position debout ; et
- un saturomètre 34 pour mesurer la saturométrie de l'utilisateur par oxygénation sanguine.

Dans le mode de réalisation décrit ici, le saturomètre 34 est fixé à l'extrémité d'un accoudoir 35 du fauteuil 30.

Conformément à l'invention, la cabine de santé 10 comporte un certain nombre de capteurs pour déterminer les conditions dans lesquelles sont effectuées les prises de mesure de santé.

Dans l'exemple de réalisation décrit ici, ces capteurs sont :
- un capteur 41 permettant de déterminer si la porte 25 de la cabine est ouverte ou fermée ;
- un capteur de pression 42 placé sur l'assise du fauteuil 30 permettant de détecter si l'utilisateur est assis ou non sur le siège ;
   - - un capteur de pression 43 placé dans le dossier du fauteuil 30, qui permet de déterminer si l'utilisateur est adossé dans le fauteuil 30 ;
- deux capteurs 44 placés sur une barre de pieds solidaire du fauteuil 30 et qui permettent de détecter si l'utilisateur a effectivement ses deux pieds en appui sur cette barre :
- deux capteurs 45 placés sous la toise 33 pour déterminer si l'utilisateur est correctement placé au moment où il mesure sa taille avec la toise 33 ou au moment où il prend sa tension en position debout avec le brassard gonflable 31 ;
- un thermomètre 46 pour mesurer la température à l'extérieur de la coque 20 ;
- un thermomètre 47 pour mesurer la température à l'intérieur de la coque 20 ;
- un hygromètre 48 pour mesurer le taux d'humidité à l'extérieur de la coque 20 ;
- un hygromètre 53 pour mesurer le taux d'humidité à l'intérieur de la coque 20 ;
- un microphone 50 pour mesurer le niveau de bruit à l'intérieur de la cabine 10 ; et
- une cellule photoélectrique 54 pour mesurer le niveau de luminosité à l'extérieur de la coque 20.

Dans le mode de réalisation décrit ici, la cabine de santé 10 comporte une lampe 55 pour diffuser, à l'intérieur de la cabine 10, une lumière d'intensité prédéterminée, de façon à ce que la luminosité de la coque 20 soit optimale pour effectuer des mesures de santé lorsque la porte 25 est fermée.

Conformément à l'invention, lorsqu'une mesure de santé est prise, les conditions dans lesquelles cette mesure a été prise sont enregistrées dans une structure de données en association avec les résultats de cette mesure.

Dans l'exemple décrit ici, cette structure de données est un fichier informatique.

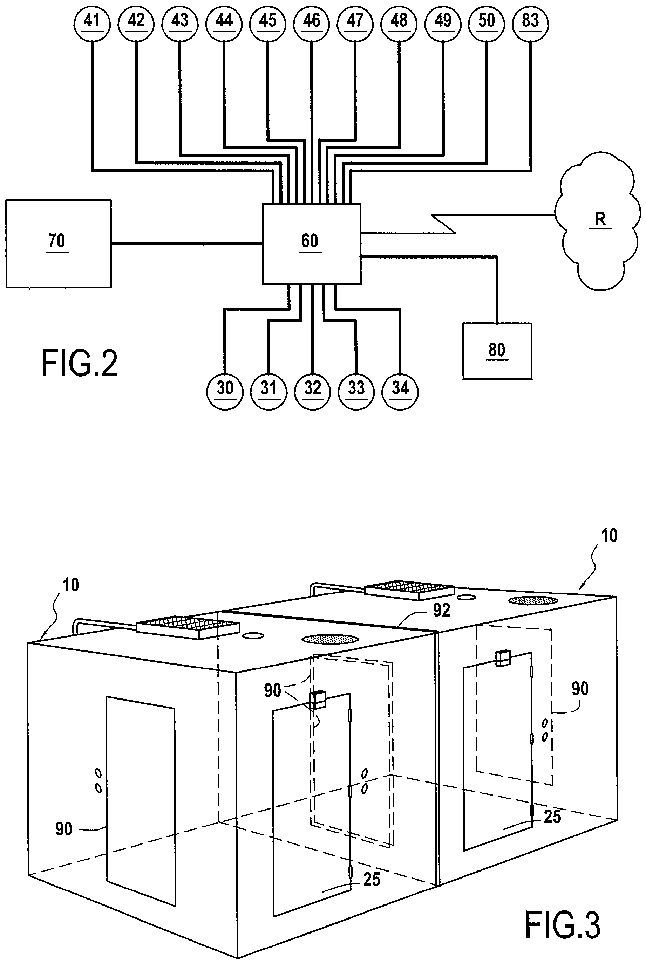

La cabine de santé 10 comporte un ordinateur 60 auquel sont reliés chacun des capteurs et des appareils de mesure précités, comme représenté à la figure 2.

Cet ordinateur 60 est adapté à générer un fichier tel que représenté à l'annexe 1 et à envoyer ce fichier à un centre distant 100 via un réseau de télécommunications R.

Dans l'exemple décrit ici, cet ordinateur 100 est installé dans l'épaisseur de la coque 20.

Dans le mode particulier de réalisation décrit ici, l'ordinateur 60 et tous les équipements électriques de la cabine de santé 10 sont alimentés en énergie par un moteur 70 placé dans l'épaisseur de la coque 20, lui-même relié à un panneau solaire 71.

De façon connue, le panneau solaire 71 réchauffe un fluide injecté dans le moteur 70 par une canalisation 72, le moteur utilisant cette énergie calorifique pour générer l'alimentation électrique nécessaire au fonctionnement des instruments électriques de la cabine 10, et pour produire du froid par compression d'un autre fluide.

Le moteur 70 est donc en particulier apte à réguler la température à l'intérieur de la cabine 20, en y injectant de l'air chaud ou moyen d'une grille 72.

La cabine de santé 10 décrite ici comporte également une grille d'aération 29 et une dalle tactile 80 permettant d'interagir avec l'utilisateur.

Nous allons maintenant décrire un scénario d'utilisation de la cabine de santé 10 pour établir le bilan de santé d'un utilisateur, ce bilant étant représenté sous forme de fichier à l'annexe 1.

Nous supposons que l'utilisateur entre dans la cabine de santé 10 par la porte 25, en laissant la porte ouverte derrière lui, ce qui est détecté par le capteur 41.

Sur la dalle tactile 80, on diffuse un message lui proposant de saisir son âge. Aussi longtemps que cette donnée n'est pas saisie, les mesures de santé ne peuvent pas commencer dans ce mode de réalisation. L'âge est ensuite enregistré dans le fichier de l'annexe 1.

Une fois l'âge de l'utilisateur saisi, un message s'affiche sur la dalle tactile, invitant l'utilisateur à mesurer sa taille avec la toise 33.

Aussi longtemps que l'utilisateur ne positionne pas ses pieds correctement sur les repères au niveau des capteurs 45, un message d'invitation lui demande de se repositionner.

La taille est ensuite enregistrée dans le fichier de l'annexe 1.

Une fois la taille mesurée, on invite l'utilisateur à rester positionné debout sur les repères précités et à effectuer une mesure de tension artérielle en position debout. Des capteurs placés dans le brassard de tension 31 permettent de détecter que le brassard est correctement positionné.

Lorsqu'une telle détection a lieu, l'ordinateur 60 déclenche un compteur et prend deux mesures de tension artérielle, respectivement au bout d'une minute et au bout de cinq minutes, les résultats de ces mesures, respectivement 11.8 et 11.6, étant mémorisés le fichier de l'annexe 1.

On invite ensuite l'utilisateur à prendre position sur le fauteuil 30.

Pour la mesure de poids, on s'assure que l'utilisateur est effectivement assis (capteur 42 actif) et que ses deux pieds sont positionnés sur la barre de pieds (position des pieds détectée par les capteurs 44).

Dès que c'est le cas, le poids est mesuré par la balance 32 et enregistré dans le fichier de l'annexe 1 (81 kg).

Un message de la dalle tactile 80 invite ensuite l'utilisateur à repositionner le brassard de tension 31 pour mesurer sa tension artérielle en position assise.

Dans le mode de réalisation décrit ici, pour que la mesure de tension artérielle soit optimisée, on souhaite que l'utilisateur soit en position assise (détecté par le capteur 42) adossé au fauteuil 30 (détecté par le capteur 43) et le bras posé sur l'accoudoir 35 (détecté par deux capteurs de position 83).

Dès que cette position est enregistrée par l'ordinateur 60, il demande à l'utilisateur de patienter 5 minutes.

Il s'avère, dans cet exemple, que, malheureusement, lorsque le délai de 5 minutes expire, l'utilisateur a retiré son bras de l'accoudoir 35.

La tension artérielle 12.7 mesurée est enregistrée dans la structure de données, avec des informations représentatives du fait que l'utilisateur était assis, adossé mais que son bras n'était pas positionné sur l'accoudoir.

Au moment de mesurer les pulsations cardiaques de l'utilisateur à l'aide du brassard de tension 31, le microphone détecte un niveau de bruit très important, à savoir un niveau de bruit de 120 dB dû à un marteau-piqueur.

Il est connu que le niveau de bruit influence fortement le nombre de pulsations cardiaques par minute.

En conséquence, la dalle tactile 80 invite l'utilisateur à fermer la porte 25, ce qui est automatiquement détecté par le capteur 41.

On enregistre alors un niveau de bruit de 50 dB à l'intérieur de la cabine de santé 10.

Lorsque la porte 25 est fermée, la lumière diffusée par la lampe 55 est au niveau optimal de 120 cd.

On supposera qu'au moment de la prise de mesure, la température interne à la cabine est de 19°C.

La dalle tactile 80 invite l'utilisateur à patienter 1 minute et les pulsations cardiaques de 60 ppm sont alors enregistrées dans le fichier de l'annexe 1.

Enfin, un message invite l'utilisateur à mesurer sa saturométrie en plaçant son index dans de saturomètre 34 positionné à l'extrémité de l'accoudoir 35.

Les mesures de luminosité (120 cd) et d'hygrométrie (4%) sont enregistrées en association avec le résultat de cette mesure de saturométrie SpO₂ : 95%.

Le fichier est ensuite automatiquement envoyé par l'ordinateur 60 à un centre distant 100 par l'intermédiaire d'un réseau de télécommunications R.

Dans l'exemple de réalisation décrit ici, la cabine de santé 10 comporte deux portes amovibles 90 situées en vis-à-vis.

Comme représenté à la figure 3, ces portes permettent de réaliser un hôpital en positionnant deux cabines de santé côte-à-côte et en retirant les panneaux amovibles 90 de façon à créer un sas de passage entre les deux cabines.

Dans un mode préféré de réalisation, les cabines de santé 10 sont reliées entre elles par un joint étanche 92 dans cet agencement particulier.

### ANNEXE 1

| | |
|---|---|
| Age : | 30 ans |

### Tension Artérielle :

| | | |
|---|---|---|
| Assis : | 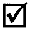 | 12.7 |
| Adossé : | 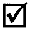 | |
| Bras sur accoudoir : | □ | |
| Durée : | 5 min | |

| DEBOUT | | |
|---|---|---|
| 1 min : | | 11.8 |
| 5 min : | | 11.6 |

### Poids :

| | | |
|---|---|---|
| Assis : | 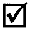 | 81 kg |
| Pieds reposés : | 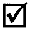 | |

| | |
|---|---|
| Taille : | 178 cm |

### Pulsations cardiaques

| | | |
|---|---|---|
| T : | 19°C | 60 ppm |
| Luminosité : | 120 cd | |
| Niveau de bruit : | 50 dB | |
| 1 min | 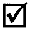 | |

### Saturométrie

| | | |
|---|---|---|
| Luminosité : | 120 cd | Sp0₂ 95% |
| Hygrométrie | 4% | |

## Revendications

1. Cabine de santé (10) comportant une coque (20), au moins un fauteuil (30), au moins un moyen de mesure (31, 32, 33, 34) d'une donnée relative à la santé d'un utilisateur,
- des moyens (41-50) de détermination pour déterminer, au moment d'une prise de mesure d'une donnée relative à la santé de l'utilisateur, au moins une condition environnementale influant sur ladite mesure, ladite condition étant nécessaire à une bonne interprétation de ladite mesure ;
- des moyens pour enregistrer, dans une structure de données, ladite au moins une condition environnementale au moment de ladite prise de mesure et le résultat de ladite mesure en association avec ladite condition, ladite structure de données comprenant, pour ladite donnée, une table d'association dont une colonne comprend le résultat de ladite mesure, et dont une autre colonne comprend ladite au moins une condition environnementale, la structure étant destinée à permettre une interprétation fiable dudit résultat par un médecin ; et
- des moyens (60) de transmission de ladite structure de données à un centre distant (100),
la cabine comportant des moyens (60) pour contrôler que ladite mesure n'est effectuée que lorsque ladite condition répond à au moins un critère prédéterminé,
dans lequel lesdits moyens (41-50) de détermination sont aptes à déterminer au moins une condition environnementale parmi :
- une position dudit utilisateur, ladite position influant sur une mesure d'une donnée relative à la santé d'un utilisateur correspondant à une mesure de la tension artérielle ;
- la température à l'intérieur et/ou à l'extérieur de ladite coque, ladite température influant sur une mesure d'une donnée relative à la santé d'un utilisateur correspondant à la mesure de tension artérielle ;
- un niveau sonore à l'intérieur et/ou à l'extérieur de ladite coque, ledit niveau sonore influant sur une mesure d'une donnée relative à la santé d'un utilisateur correspondant à la mesure de tension artérielle ;
- un taux d'humidité à l'intérieur et/ou à l'extérieur de ladite coque, ledit taux d'humidité influant sur une mesure d'une donnée relative à la santé d'un utilisateur correspondant à une mesure de saturométrie par oxygénation sanguine ; et
- un niveau de luminosité à l'intérieur et/ou à l'extérieur de ladite coque, ledit niveau de luminosité influant sur une mesure d'une donnée relative à la santé d'un utilisateur correspondant à la mesure de saturométrie par oxygénation sanguine.

2. Cabine de santé selon la revendication 1, **caractérisée en ce que** ladite coque (20) comporte au moins une ouverture (90) permettant de relier ladite cabine de santé à au moins une autre cabine de santé.

3. Cabine de santé selon la revendication 1 ou 2, **caractérisée en ce qu'**elle comporte des moyens (70) d'alimentation en énergie.

4. Système de santé comprenant :
- une cabine de santé selon l'une quelconque des revendications 1 à 3 ;
- un centre distant (100),
ladite cabine de santé et ledit centre distant étant connectés via un réseau de télécommunications.

## Patentansprüche

1. Gesundheitskabine (10), umfassend eine Hülle (20), mindestens einen Stuhl (30), mindestens eine Messeinrichtung (31, 32, 33, 34) von Daten in Bezug auf die Gesundheit eines Benutzers,
- Bestimmungseinrichtungen (41-50), um, zum Zeitpunkt einer Messung von Daten in Bezug auf die Gesundheit des Benutzers, mindestens eine Umgebungsbedingung zu bestimmen, die die Messung beeinflusst, wobei die Bedingung für eine korrekte Interpretation der Messung erforderlich ist;
- Einrichtungen zum Registrieren, in einer Datenstruktur, der mindestens einen Umgebungsbedingung zum Zeitpunkt der Messung und des Resultats der Messung in Assoziation mit der Bedingung, die Datenstruktur umfassend, für die Daten, eine Assoziationsstabelle, deren eine Spalte das Resultat der Messung umfasst, und deren andere Spalte die mindestens eine Umgebungsbedingung umfasst, wobei die Struktur dazu bestimmt ist, eine zuverlässige Interpretation des Resultats durch einen Arzt zu ermöglichen; und
- Einrichtungen (60) zur Übertragung der Datenstruktur an ein entferntes Zentrum (100),
die Kabine umfassend (60) Einrichtungen, um zu steuern, dass die Messung nur dann ausgeführt wird, wenn die Bedingung mindestens ein vorbestimmtes Kriterium erfüllt,
wobei die Einrichtungen (41-50) zur Bestimmung geeignet sind, um mindestens eine der Umgebungsbedingungen zu bestimmen:
- eine Position des Benutzers, wobei die Position eine Messung von Daten in Bezug auf die Gesundheit eines Benutzers beeinflusst, die einer Messung des Blutdrucks entspricht;
- die Temperatur innerhalb und/oder außerhalb der Hülle, wobei die Temperatur eine Messung von Daten in Bezug auf die Gesundheit eines Benutzers beeinflusst, die der Blutdruckmessung entspricht;
- einen Schallpegel innerhalb und/oder außerhalb der Hülle, wobei der Schallpegel eine Messung von Daten in Bezug auf die Gesundheit eines Benutzers beeinflusst, die der Messung des Blutdrucks entspricht;
- einen Feuchtigkeitsgehalt innerhalb und/oder außerhalb der Hülle, wobei der Feuchtigkeitsgehalt eine Messung von Daten in Bezug auf die Gesundheit eines Benutzers beeinflusst, die einer Messung von Sauerstoffsättigung des Bluts entspricht; und
- ein Helligkeitsniveau innerhalb und/oder außerhalb der Hülle, wobei das Helligkeitsniveau eine Messung von Daten in Bezug auf die Gesundheit eines Benutzers beeinflusst, die der Messung von Sauerstoffsättigung des Bluts entspricht.

2. Gesundheitskabine nach Anspruch 1, **dadurch gekennzeichnet, dass** die Hülle (20) mindestens eine Öffnung (90) umfasst, die es ermöglicht, die Gesundheitskabine mit mindestens einer anderen Gesundheitskabine zu verbinden.

3. Gesundheitskabine nach Anspruch 1 oder 2, **dadurch gekennzeichnet, dass** sie Einrichtungen (70) zur Energieversorgung umfasst.

4. Gesundheitssystem, umfassend:
- eine Gesundheitskabine nach einem der Ansprüche 1 bis 3;
- ein entferntes Zentrum (100),
wobei die Gesundheitskabine und das entfernte Zentrum über ein Telekommunikationsnetz miteinander verbunden sind.

## Claims

1. A health booth (10) including a shell (20), at least one chair (30), at least one measuring means (31, 32, 33, 34) for measuring a datum relative to the health of a user,
- determining means (41-50) for determining, at the time of measurement of a datum relative to the health of a user, at least one environmental condition influencing said measurement, said condition being necessary for a proper interpretation of said measurement;
- means for recording, in a data structure, said at least one environmental condition at the time of said measurement and the result of said measurement in association with said condition, said data structure comprising, for said datum, a match table whereof one column comprises the result of said measurement, and whereof another column comprises said at least one environmental condition, the structure being intended to allow a reliable interpretation of said result by a physician; and
- transmission means (60) for transmitting said data structure to a remote center (100),
the booth including means (60) for checking that said measurement is done only when said condition meets at least one predetermined criterion,
wherein said determining means (41-50) are able to determine at least one environmental condition among:
- a position of said user, said position influencing a measurement of a datum relative to the health of a user corresponding to a measurement of the blood pressure;
- the temperature inside and/or outside said shell, said temperature influencing a measurement of a datum relative to the health of the user corresponding to the blood pressure measurement;
- a sound level inside and/or outside said shell, said sound level influencing a measurement of a datum relative to the health of a user corresponding to the blood pressure measurement;
- a humidity level inside and/or outside said shell, said humidity level influencing a measurement of a datum relative to the health of the user corresponding to a blood oxygen saturation measurement; and
- a brightness level inside and/or outside said shell, said brightness level influencing a measurement of a datum relative to the health of the user corresponding to the blood oxygen saturation measurement.

2. The health booth according to claim 1, **characterized in that** said shell (20) includes at least one opening (90) allowing said health booth to be connected to at least one other health booth.

3. The health booth according to claim 1 or 2, **characterized in that** it includes power supply means (70).

4. A health system, comprising:
- a health booth according to any one of claims 1 to 3;
- a remote center (100),
said health booth and said remote center being connected via a telecommunication network.
